# EUROPÄISCHE PATENTANMELDUNG

(11) **EP 0 992 257 A1**
(43) Veröffentlichungstag der Anmeldung: **12.04.2000**
(21) Anmeldenummer: 99119613.0
(22) Anmeldetag: 04.10.1999
(51) Int. Cl.: A61M 5/14, A61M 5/38, A61M 39/02

(54) **Infusionssammler**

(30) Priorität: 05.10.1998 DE 29817718 U
(71) Anmelder: CareMed Medical Produkte Aktiengesellschaft, 01109 Dresden (DE)
(72) Erfinder: Heise, Peter, D-34277 Fuldabrück (DE)
(74) Vertreter: Hofmann, Klaus, Dipl.-Ing.

(57) **Zusammenfassung**

Die Erfindung betrifft einen Infusionssammler einfacher Bauart und mit sehr hoher Sicherheit für den Patienten bezüglich unbekannter oder ungewollter Unterbrechungen der Infusion bzw. der Dosierung der einzelnen Infusionsflüssigkeiten im Bolus. Dies wird dadurch erreicht, daß der Infusionssammler zwei Gehäuseteile aufweist, die durch Schweißen, Kleben, Kraftschluß oder Formschluß miteinander verbunden sind. Auf dem oberen Gehäuseteil sind Aufsätze mit Anschlüssen für die Infusionsleitungen angeordnet. In den Aufsätzen sind Rückschlagventile angeordnet, die durch Schwimmergehäuse, Druckfederkräfte oder gummielastische bzw. plastelastische Kräfte betätigt werden.

Am Abgang sind im Infusionssammler je ein hydrophiles und ein hydrophobes Filter im Abstand voneinander angeordnet, so daß feste Partikel bzw. Luft- oder Gasblasen vom Patienten ferngehalten werden. Zur Vermeidung des Abknickens der Schlauchleitungen sind an einem Gehäuseteil gegenüber der Trägerplatte Krallen zur Schlauchführung angeordnet.

## Beschreibung

Die Erfindung betrifft einen Infusionssammler, d.h. eine Vorrichtung, die dem Zusammenführen und Mischen mehrerer Infusions- und Injektionsflüssigkeiten, die aus Spritzen, Schwerkraftinfusionsvorrichtungen und Perfusionspumpen stammen, dient. Die einzelnen Rückschlagventile, die den einzelnen Infusionsleitungen zugeordnet sind, haben die Aufgabe, bei Störungen des Abflusses aus dem Infusionssammler, einen Rückfluß in eine Zuleitung niedrigeren Druckes aus einer Zuleitung höheren Druckes zu verhindern.

Am Ausgang des Infusionssammlers befindet sich ein hydrophobes Luftfilter zum Abscheiden eventuell eingeschlossener Luftblasen, die das Leben des Patienten bedrohen könnten. Das hydrophile Filter hält eventuell in der Infusionsflüssigkeit vorhandene Partikel zurück.

Stand der Technik und Nachteile des Standes der Technik: In der DE 40 04 134 AI ist ein Infusionssammler beschrieben, der ein von den Infusionsflüssigkeiten durchströmtes Gehäuse mit mehreren jeweils separat verschließbaren Zugängen sowie einem Abgang für die zusammengeführten Flüssigkeiten aufweist. Die Zugänge sind abgewinkelt und münden in einer gemeinsamen Ebene. Ein im Querschnitt winkelförmiges elastisches Dichtelement ist mit seinem senkrecht zu der genannten Ebene verlaufenden Schenkel gehalten, während der andere Schenkel mit Federdruck in der genannten Fläche gegen die Mündungen der Zugänge drückt und diese verschließt. Auf diese Weise wird für jeden Zugang ein Rückschlagventil gebildet, das den Eintritt einer Infusionsflüssigkeit aus einem Zugang in einen anderen Zugang verhindert.

Dieser bekannte Infusionssammler hat gleich mehrere Nachteile. Zunächst ist er äußerst kompliziert in seinem Aufbau. Das im Querschnitt winkelförmige Dichtelement muß genau und dicht eingespannt sein. Darüber hinaus muß es mit seinem dichtenden Schenkel in der Ebene, in der die Zugänge münden, dicht an der Mündungsfläche anliegen. Da dabei die Anlagekraft der durch eine Schenkelbewegung der dichtenden Schenkel des Dichtelements erzeugt wird, ist es außerordentlich schwierig, eine vollständige Dichtung rund um den Mündungsbereich eines Zuganges, und dies auch für alle Zugänge zu erreichen. Dies kann außerdem nur für den Fall sein, wenn die Anlagekraft verhältnismäßig groß ist. Dann muß aber auch der Öffnungsdruck verhältnismäßig groß sein. Aus diesen Gründen ist der bekannte Infusionssammler für Schwerkraftinfusionen nicht geeignet.

Durch das DE GM 74 22 657 ist ein Infusionssammler bekannt, bei dem je Zuleitung ein seitlicher Innenkonus zum Ansetzen eines Verbindungsstückes mit Außenkonus vorgesehen ist und bei dem im Bereich des seitlichen Anschlußstückes elastische, im Normalzustand an der Innenwandung des Infusionssammlers anliegende Schlauchstücke angeordnet sind.

Der Innenkonus besitzt eine solche Weite, daß die Stirnseite des Außenkonus des Verbindungsstückes in eingesteckter Lage das Schlauchstück aus seiner abdichtenden Lage in die Offenstellung drückt. Das elastische Schlauchstück soll dabei auch als Rückschlagventil wirken und sich zeitweilig öffnen, wenn mit Hilfe einer Injektionsspritze ein entsprechend hoher Injektionsdruck erzeugt wird.

Das Zuführen einer Injektionslösung setzt also voraus, daß ein entsprechend hoher Druck in der Injektionsleitung erzeugt werden kann. Damit ist der Anwendungsbereich dieses bekannten Infusionssammlers außerordentlich begrenzt, und er ist nicht für Schwerkraftinfusionsgeräte geeignet. Ist das Verbindungsstück außerdem als Mehrfachverbindungsstück ausgebildet, so hat es einen komplizierten Aufbau, bei dem an einem zentralen, längeren Rohrstück mehrere Anschlüsse für mehrere Infusionsleitungen vorgesehen sind. Dadurch ergibt sich ein kompliziertes, unübersichtliches und langes Gebilde. Die DE 43 25 837 A1 beschreibt einen InfusionsSammler, der aus einer ringförmigen Kammer besteht, in die mehrere Anschlußkanäle münden. Die Abdichtung der einzelnen Anschlußkanäle erfolgt durch einen kurzen Schlauchabschnitt 6, der die Wirkung eines Rückschlagventils hat. Zur Vermeidung eines Übertritts der Infusionsflüssigkeit von einem Anschlußkanal zum anderen sind Ausnehmungen 15 vorgesehen, über die die Infusionsflüssigkeit in die Kammer 4 abfließen kann. Der kurze Schlauchabschnitt 6 ist im Querschnitt ballig ausgebildet, damit bei einem Überdruck die Infusionsflüssigkeit über den dünneren Rand in die Kammer 4 entweichen kann.

Ein Überdruck entsteht dann, wenn die Infusionsleitung zwischen der Abflußleitung 9 und dem Patienten z.B. abgeknickt ist. Die Schwerkraftinfusionen kommen in diesem Fall zum Stillstand, während die Infusionspumpen weiter arbeiten. Aufgrund der Elastizität des ableitenden Systems werden sich also die ringförmige Kammer 4, die Ausnehmungen 15, der scheibenförmige Raum 8, der Verbindungskanal 11, die Abflußleitung 9 und gegebenenfalls die Infusionsleitung zum Patienten (teilweise) mit dem hochwirksamen Medikament aus der Infusionspumpe auffüllen.

Nach Beseitigung des Hindernisses im Schlauch zum Patienten wird dieses hochwirksame Medikament dem Patienten im Bolus zugeführt, der dadurch stark gefährdet ist.

Wird das Hindernis im Schlauch zum Patienten nicht beseitigt und die Infusionspumpe schaltet nicht selbständig ab, wird trotz der balligen Form des kurzen Schlauchabschnittes 6 das hochwirksame Medikament von einem Anschlußkanal 2 in einen anderen gepumpt. Damit ist die Schutzfunktion des Sicherheitsverbinders nicht gewährleistet.

In der DE 43 07 203 C1 wird ein Überdruckventil (Fahrradventil) beschrieben, dessen Aufgabe darin besteht, einen so hohen Öffnungsdruck aufzubauen, daß der am Ventil lastende Unterdruck einerseits bei einer angeschlossenen Medikamentenspritze das Medikament nicht unbeabsichtigt zum Patienten strömen läßt und andererseits nach Abtrennung der Spritze keine Luft in das Schlauchsystem eintreten läßt bzw. eingesaugt werden kann.

Für diesen Anwendungsfall, in dem Flüssigkeit mittels einer Pumpe von der Zuleitung 6 in die Ableitung 16 gepumpt wird, ist der Druckverlust Δp über das Schlauchstück (Ventil) 10 von untergeordneter Bedeutung.

In Anwendungsfällen, wo die Druckdifferenz Δp einen Wert von annähernd Null erreichen soll, ist diese Anordnung aufgrund der hohen Radialspannungen im Schlauch 9 nicht einsetzbar.

Weiterhin weist das Überdruckventil konstruktiv bedingt Strömungsumlenkungen und damit Totwassergebiete auf. In diesen Totwassergebieten ist bei auftretenden bakteriellen Verunreinigungen eine sehr schnelle und intensive Bakterienvermehrung möglich. Ablagerungen von Mineralien (sogenannten Inkrustationen) aus der durch das Ventil strömenden Flüssigkeit sind in diesen Stagnationsgebieten unvermeidlich.

In dem DE GM 85 16 350 ist eine Anordnung zur Mischung und Verabreichung verschiedener Infusionslösungen dargestellt, bei der gleichzeitig Infusionen über Schwerkraft und Pumpen erfolgen können. Zusätzlich sind Injektions- und Entnahmemöglichkeiten vorgesehen.

Dieser bekannte Infusionssammler hat mehrere Nachteile, die die Gesundheit und das Leben des Patienten gefährden. Aufgrund der gewählten Anordnung sind nur Rückschlagventile mit einer kleinen Dichtfläche - wie die angegebenen Schnabelventile (oder z.B. Kugel- oder Plattenventile) - einsetzbar. Bei zähflüssigen Lösungen ist eine Verklebung und bei mineralhaltigen Lösungen eine Inkrustation der Dichtfläche nicht auszuschließen. Gibt man den Ventilen eine höhere Vorspannung, erhöht sich der Öffnungsdruck. Beide Phänomene führen zu einer Disfunktion. Entweder öffnet das Ventil nicht richtig, dann wird eine Infusionslösung unterdosiert, oder durch ein undichtes Ventil strömt unter Umständen ein hochdosiertes Medikament zurück, das zu einem späteren Zeitpunkt dem Patienten im Bolus verabreicht wird und damit ausgesprochen gefährliche Wirkungen haben kann.

Die Montage der Ventile erscheint sehr kompliziert und zeitaufwendig, was hohe Kosten für die Produkte verursacht.

Ein Entlüftungsfilter ist nicht vorgesehen, was den Patienten ebenfalls gefährden kann (Luftembolie). Ebenso fehlt ein Partikelfilter, d.h. alle korpuskulären Bestandteile gelangen ungehindert in den Kreislauf des Patienten.

In dem USP 54 31 185 ist ein Infusionssammler mit Zuspritzport beschrieben, bei dem zwischen zwei Gehäuseteilen eine Membran 20 eingelegt ist, die die Zuströmöffnungen der einzelnen Infusionen abdeckt und damit einen Reflux verhindert.

Als Nachteil dieses bekannten Infusionssammlers ist die Konstruktion der Nocken 30a-c anzusehen, die eine Deformation der abdeckenden Membran hervorruft und damit eine sehr genaue Abstimmung von Geometrie und Material erfordert, um eine Dichtigkeit gegen Reflux zu erreichen.

Der Distanzhalter 26a,b und die Nockenpaare 34a,b sind ebenfalls sehr genau zu bemessen und müssen mit der Membran 20 abgestimmt sein, um einen Flüssigkeitsaustausch zwischen den Zulaufkanälen 24a-c zu verhindern.

Da es sich bei dem Infusionssammler um einen Massenartikel handelt, ist es technisch sehr aufwendig, für beide möglichen Funktionsstörungen eine geeignete Lösung zu finden. Die Nachteile der bekannten Infusionssammler bestehen zusammengefaßt darin, daß die verwendeten Rückschlagventile zu langsam ansprechen oder bei geringen Drücken nicht schnell genug oder nicht absolut dicht schließen, so daß die hochwirksamen Medikamente aus dem Perfusionssystem entweder diskontinuierlich (ohne Verdünnung aus den Schwerkraftsystemen) oder im Bolus (nach Rückfluß in ein Schwerkraftsystem) zum Patienten gelangen können. In beiden Fällen ist eine Gesundheitsgefährdung der meist schwerstkranken Patienten nicht auszuschließen.

Weiterhin sind die bekannten Ausführungen teilweise sehr aufwendig konstruiert, so daß hohe Herstellkosten und auch voluminöse Produkte daraus resultieren, die nur aufwendig zu handeln und zu entsorgen sind.

Die Aufgabe der vorliegenden Erfindung ist es, unter Vermeidung der Nachteile des Standes der Technik einen Infusionssammler zu schaffen, dessen Konstruktion so anzulegen ist, daß das Endprodukt kompakt und handlich ist und zusätzlich zu- und abführende Schlauchleitungen sicher vor einem Abknicken geschützt sind und Ordnung rund um das Patientenbett herrscht, um dem Pflegepersonal ein einfaches Arbeiten zu ermöglichen. Die verwendeten Materialien sind so auszuwählen, daß eine umweltgerechte Entsorgung der Produkte gewährleistet werden kann.

Es sind Rückschlagventile zu konzipieren, die bei einer minimalen Druckänderung im Inneren des Infusionssammlers sofort ansprechen und einen dauerhaften Verschluß der Niederdruckzuleitungen gewährleisten.

Die Aufgabe wird durch die kennzeichnenden Merkmale des Anspruchs 1 gelöst. Ausgestaltende Merkmale sind in Anspruch 2 beschrieben. Erfindungsgemäß sind die Rückschlagventile über Schwimmergehäuse oder über DruckfederKörper oder über ummielastische Körper oder über plastelastische Körper angetrieben. Der Infusionssammler besteht aus einem unteren Gehäuseteil und aus einem oberen Gehäuseteil. Beide Gehäuseteile sind durch Schweißen oder Kleben oder Kraft- oder Formschluß miteinander verbunden. Gegenüberliegend an den Gehäuseteilen sind ein Halter für die Befestigung am Infusionsstativ und Krallen für die knickfreie Führung und Halterung der Schlauchleitungen, die die einzelnen Flüssigkeiten dem Infusionssammler über Aufsätze mit Anschlüssen zuführen. Vor dem Abgang zum Patienten sind im Infusionssammler im Abstand voneinander je ein hydrophiler und ein hydrophober Filter angeordnet. Beide Filter dienen dem Schutz der schwerstkranken Patienten vor Luft- oder materiellen Einschlüssen im Infusionsgemisch. Das im oberen Gehäuseteil angeordnete hydrophobe Filter grenzt gehäuseseitig an einen über Gehäusedurchbrüche mit der Atmosphäre in Verbindung stehenden Raum, so daß eventuelle Luft- oder Gaseinschlüsse entweichen können.

Die erfindungsgemäße Lösung hat den Vorteil, daß unter Vermeidung der Nachteile des Standes der Technik ein schnelles und zuverlässiges Schließen der Rückschlagventile möglich ist. Sogenannte Kriechflüsse der Infusionsflüssigkeit sind ausgeschlossen. Die Bauweise ist kompakt und leicht handhabbar. Die Befestigung am Infusionsständer ist sicher gewährleistet und die Ordnung am Patientenbett (keine abknickenden und herumhängenden Infusionsschläuche) ist gesichert. Ebenso besteht eine hohe Sicherheit für das Leben des Patienten durch Vermeidung der Unterbrechung der Infusion oder durch gravierende Veränderungen der Dosierung.

### Ausführungsbeispiel

Anhand eines Ausführungsbeispiels und der Zeichnung soll die Erfindung näher erläutert werden.

Es zeigen:
- Fig. 1:: einen Schnitt durch den erfindungsgemäßen Infusionssammler;
- Fig.2:: eine Draufsicht des geschlossenen Infusionsfilters, wie er am Infusionsstativ verwendet wird;
- Fig.3:: einen Schnitt durch einen Aufsatz mit Schnitt durch ein mittels Schwimmergehäuse angetriebenen Rückschlagventils;
- Fig.4:: einen Schnitt gemäß Fig.3, jedoch mit Druckfederkraft angetriebenen Rückschlagventil;
- Fig.5:: einen Schnitt gemäß Fig.3 mit gummielastischem Antrieb des Rückschlagventils.

Der erfindungsgemäße Infusionssammler besteht aus zwei Gehäuseteilen 7a und 7b, die in an sich bekannter Weise durch Schweißen, Kleben, Kraftschluß oder Formschluß miteinander verbunden sind. Das obere Gehäuseteil 7b ist mit Aufsätzen 8, in denen die Rückschlagventile angeordnet sind, und mit Anschlüssen 9 für die Zuläufe 1 versehen. Die Anzahl der Aufsätze 8 kann nach Bedarf beliebig groß sein, vorzugsweise 4 oder 6. An dem Gehäuseteil 7a oder 7b ist eine Trägerplatte 10a, die der Befestigung des Infusionssammlers an einem Infusionsständer dient, angeordnet. Weiterhin sind an dem Gehäuseteil 7a oder 7b, jedoch an entgegengesetzter Seite der Trägerplatte 10a Krallen 10b angeordnet. Sie dienen der Aufnahme der Infusionsschläuche und verhindern ein Abknicken derselben.

Die zugeführten Flüssigkeiten verlassen den Infusionssammler über ein hydrophiles Filter 6 und den Abgang 2. Eventuell in der Flüssigkeit befindliche Lufteinschlüsse werden über das hydrophobe Filter 5 in die Umgebung ausgeschieden. Dieses hydrophobe Filter ist durch kleine Gehäusedurchbrüche 11 und einen freien Raum 12 oberhalb des Filters 5 vor Kontamination geschützt.

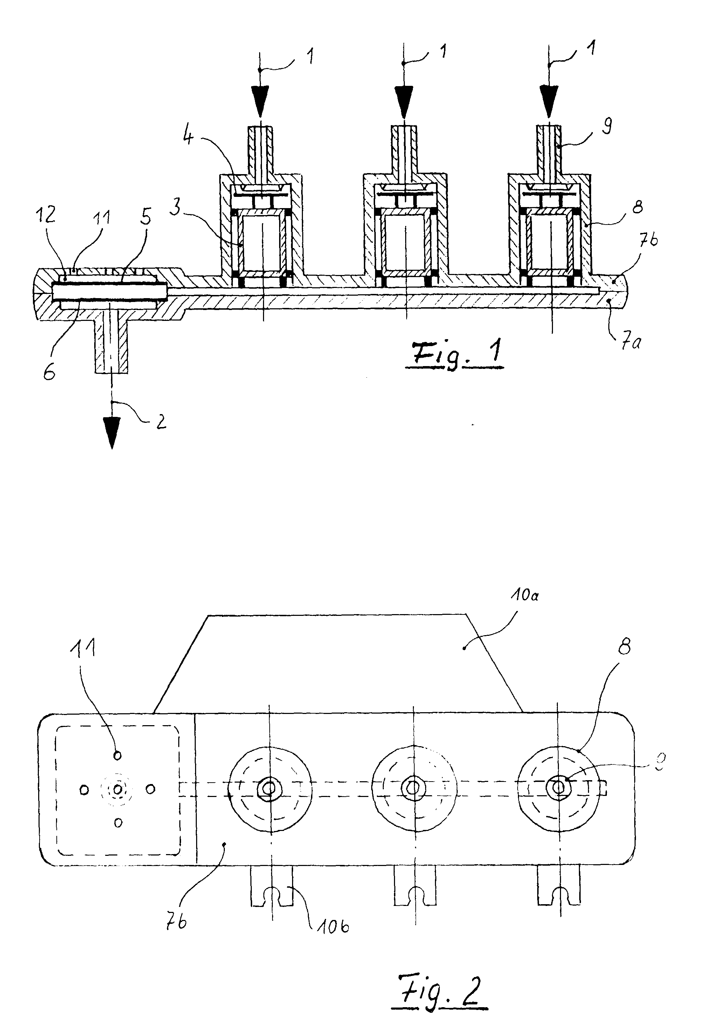

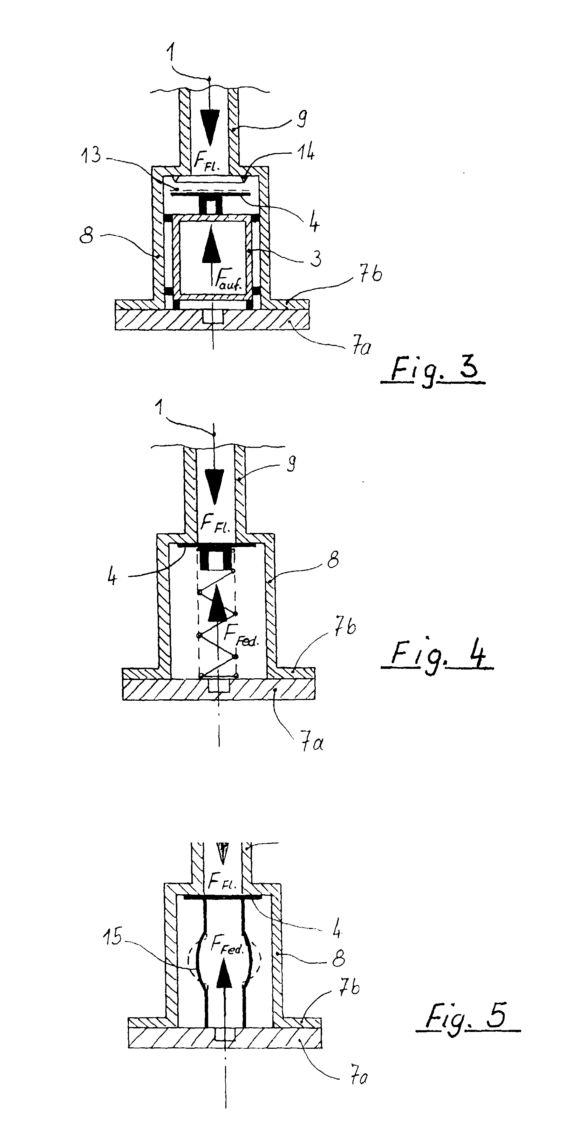

Das Rückschlagventil kann nach dem archimedischen Prinzip des Auftriebes seine Funktion erfüllen (vgl. Fig.3). Während des normalen Betriebes einer Infusion überwiegen die Flüssigkeitskräfte F_{fl} der Infusionsflüssigkeit gegenüber den Auftriebskräften F_{auf} des Schwimmergehäuses 3. Der Weg für die Infusionsflüssigkeit ist frei. Im Falle einer Störung des Abflusses am Abgang 2 ändern sich die Druckverhältnisse im Inneren des Aufsatzes 8 und das Schwimmergehäuse 13 schwimmt sofort auf, was zu einer Abdichtung des Ventiltellers 4 an der Dichtfläche 13 führt. Ein Rückfluß in den Zulauf 1 ist somit sicher verhindert.

Andere Möglichkeiten der Vermeidung eines Rückflusses gibt es, statt der Auftriebskraft F_{auf} die Kraft einer Metall- oder Kunststoffdruckfeder F_{fed} (vgl. Fig.4) oder eines gummielastischen Elements (vgl. Fig.5) auszunutzen. In allen Fällen ist eine sichere Dichtung zwischen dem Ventilteller 4 und der Dichtfläche 13 gewährleistet. Dabei ist es möglich, die Dichtfläche 13 durch eine erhabene umlaufende Wulst 14 den gegebenen Bedingungen anzupassen.

Eine weitere Möglichkeit des Antriebes des Ventiltellers 4 ist in Fig.5 dargestellt. Hier wird unterhalb des Ventiltellers 4 ein federelastisches Schlauchstück 15 angeordnet. Das Schlauchstück kann sowohl aus einem gummielastischen als auch aus einem plastelastischen Material bestehen. Durch den Druck der Infusionsflüssigkeit wird der Ventilteller 4 geöffnet und die betreffende angeschlossene Infusionsflüssigkeit kann in den Infusionssammler fließen. Dadurch baucht das Schlauchstück 15 aus. Sobald sich der Flüssigkeitsdruck im Infusionssammler über die Flüssigkeitskraft F_{fl} erhöht, wird der Ventilteller 4 zuverlässig geschlossen.

### Aufstellung der verwendeten Bezugszeichen

- 1: Zulauf
- 2: Abgang
- 3: Schwimmergehäuse
- 4: Ventilteller
- 5: hydrophobes Filter
- 6: hydrophiles Filter
- 7a: Gehäuseteil
- 7b: Gehäuseteil
- 8: Aufsatz
- 9: Anschluß
- 10a: Trägerplatte
- 10b: Kralle
- 11: Gehäusedurchbrüche
- 12: Raum
- 13: Dichtfläche
- 14: Wulst
- 15: Schlauchstück

- F_{auf}: Auftriebskraft
- F_{fl}: Flüssigkeitskraft
- F_{fed}: Federkraft

## Patentansprüche

1. Infusionssammler, bestehend aus zwei Gehäuseteilen mit mehreren Zugängen für verschiedene Infusionsflüssigkeiten und einem gemeinsamen Abgang zum Patienten und der weiterhin mit einer Trägerplatte und Aufsätzen zur Aufnahme von Rückschlagventilen versehen ist, dadurch gekennzeichnet, daß die Rückschlagventile über Schwimmergehäuse (3) oder Druckfederkörper (F_{fed}) oder gummielastische Körper oder plastelastische Körper angetrieben sind und daß zur knickfreien Halterung der Zuleitungen für die verschiedenen Infusionsflüssigkeiten am Infusionssammler Krallen (10b) angeordnet sind und daß vor dem Abgang (2) zum Patienten im Abstand voneinander je ein hydrophiles Filter (6) und ein hydrophobes Filter (5) und im oberen Gehäuseteil (7b) ein über Gehäusedurchbrüche (11) mit der Atmosphäre in Verbindung stehender freier Raum (12) angeordnet ist.

2. Infusionssammler nach Anspruch 1, dadurch gekennzeichnet, daß der Infusionssammler aus einem unteren Gehäuseteil (7a) und aus einem oberen Gehäuseteil (7b) besteht, die durch Schweißen, Kleben, Kraftschluß oder Formschluß miteinander verbunden sind.
